# FASCICULE DE BREVET EUROPEEN

(11) **EP 3 523 211 B1**
(45) Date de publication et mention de la délivrance du brevet: **01.07.2020**
(21) Numéro de dépôt: 17786980.7
(22) Date de dépôt: 05.10.2017
(51) Int. Cl.: B65D 25/10, A61F 2/00, B65D 83/00

(54) **DISPOSITIF DE CONDITIONNEMENT D'OBJET ET PROCEDE D'EXTRACTION CORRESPONDANT**
VORRICHTUNG ZUM VERPACKEN EINES OBJEKTS UND ENTSPRECHENDES VERFAHREN ZUR EXTRAKTION
DEVICE FOR PACKAGING AN OBJECT AND CORRESPONDING METHOD OF EXTRACTION

(30) Priorité: 06.10.2016 FR 1659654
(43) Date de publication de la demande: 14.08.2019
(73) Titulaire: Selenium Medical, 17000 La Rochelle (FR)
(72) Inventeur: RICHART, Olivier, 17580 Le Bois Plage en Re (FR)
(74) Mandataire: Dutreix, Hugues Ours
(86) Numéro de dépôt international: PCT/FR2017/052734
(87) Numéro de publication internationale: WO 2018/065733

(56) Documents cités:
- FR-A1- 2 959 216
- US-A1- 2003 214 139

## Description

### DOMAINE DE L'INVENTION

La présente invention concerne de manière générale le conditionnement d'objet, en particulier le conditionnement d'un implant médical.

### ART ANTERIEUR

Il est connu de l'état de la technique, notamment du document FR 2959216 A1, de conditionner un implant médical en le positionnant à l'intérieur d'un tube fermé par un bouchon. En particulier, il est connu que le bouchon soit muni d'un système d'accrochage de l'implant. L'opérateur peut ainsi retirer le bouchon de manière à extraire l'implant du tube. Puis l'opérateur désactive le système d'accrochage de l'implant pour pouvoir le saisir et l'utiliser.

Cependant, on constate que ces systèmes d'accrochage connus de l'état de la technique sont coûteux à fabriquer et/ou peu pratiques à manipuler par l'opérateur. En outre ces systèmes d'accrochage connus de l'état de la technique peuvent masquer une partie de l'implant, ce qui peut être source d'erreur ou de complication pour l'opérateur qui souhaite pouvoir identifier visuellement et rapidement l'implant contenu dans le tube.

La présente invention a pour but de proposer un nouveau dispositif et procédé de conditionnement permettant de palier à tout ou partie des problèmes exposés ci-dessus.

### RESUME DE L'INVENTION

A cet effet, l'invention a pour objet un dispositif de conditionnement d'objet comprenant :
- un tube qui est ouvert à une extrémité ;
- un bouchon permettant de refermer ladite ouverture du tube,
- un objet, tel qu'un implant médical,
caractérisé en ce que le dispositif de conditionnement comprend un élément allongé flexible, appelé bandelette,
ladite bandelette étant de longueur supérieure à la longueur du tube, ladite bandelette comprenant un système de couplage permettant de coupler ledit objet à ladite bandelette,
la bandelette étant configurée avec le tube pour prendre :
- une première position dans laquelle la bandelette est maintenue comprimée à l'intérieur du tube par le bouchon, ladite bandelette présentant une première extrémité en contact d'appui avec le fond du tube opposé à ladite ouverture,
- une deuxième position dans laquelle, le bouchon étant retiré, la bandelette présente une deuxième extrémité, opposée à ladite première extrémité, qui s'étend en saillie de l'ouverture du tube.

La bandelette est ainsi suffisamment longue pour pouvoir présenter une extrémité en saillie du tube à l'état retiré du bouchon, ce qui facilite sa saisie, mais aussi pour venir en appui par son autre extrémité contre le fond du tube, qui forme ainsi un appui pour l'extrémité de la bandelette, ce qui évite que la bandelette soit coincée dans le tube au niveau de l'objet avec lequel elle est couplée, ce qui pourrait être le cas si sa longueur était insuffisante pour toucher le fond du tube.

L'appui de l'extrémité de la bandelette sur le fond du tube permet de maintenir l'objet qui est couplé à la bandelette à une certaine hauteur par rapport au tube. La compression appliquée par le bouchon sur la bandelette permet de contraindre la bandelette et donc de limiter, voire empêcher, le mouvement de la bandelette et donc de l'objet par rapport au tube, tout en bénéficiant de l'effort de rappel qui tend à ramener la bandelette en position détendue lors de l'ouverture du bouchon et s'oppose à un coincement de l'objet dans le tube. Selon une caractéristique avantageuse de l'invention, le tube est translucide ou transparent.

Le fait de combiner l'utilisation d'une bandelette à laquelle est couplé l'objet et d'un tube transparent ou translucide, permet à l'utilisateur de visualiser aisément l'objet qui est maintenu à une hauteur donnée du tube, par exemple à mi-hauteur du tube. L'utilisateur peut ainsi rapidement et de manière fiable identifier l'objet contenu dans le tube avant même que le tube soit ouvert.

Selon une caractéristique avantageuse de l'invention, la bandelette est translucide ou transparente.

Selon une caractéristique avantageuse de l'invention, le système de couplage comprend un élément encliquetable avec un orifice ménagé dans l'objet.

Selon une caractéristique avantageuse de l'invention, ledit système de couplage comprend une portion élargie de la bandelette, située de préférence au milieu de la bandelette, qui forme une base, deux oreilles étant formées par découpe de ladite base, lesdites oreilles étant aptes à être redressées par rapport à ladite base, en étant ramenées l'une contre l'autre, pour être passées à travers un orifice ménagé dans l'objet, lesdites oreilles étant aptes à reprendre une position écartée l'une de l'autre après la traversée de l'orifice de l'objet, dans laquelle la possibilité de retrait de l'objet est limitée.

Selon une caractéristique avantageuse de l'invention, ledit système de couplage comprend deux fentes parallèles écartées ménagées à travers la bandelette pour former un passant d'insertion d'un objet, tel qu'une vis.

Selon une caractéristique avantageuse de l'invention, la bandelette se présente sous la forme d'une réglette présentant une série de crans et le système de couplage comprend deux coulisseaux rapprochables l'un de l'autre le long de la réglette pour enserrer l'objet, les crans étant configurés pour empêcher un déplacement à écartement desdits coulisseaux l'un par rapport à l'autre.

Selon une caractéristique avantageuse de l'invention, la forme du tube et celle de la bandelette sont configurées pour limiter ou empêcher la rotation de la bandelette par rapport au tube.

Selon une caractéristique avantageuse de l'invention, ledit dispositif comprend ledit objet, ledit objet étant couplé à la bandelette et contenu dans le tube.

Selon une caractéristique avantageuse de l'invention, un premier orifice et un deuxième orifice sont ménagés dans l'objet pour permettre le passage d'une portion d'extrémité de la bandelette à travers le premier orifice de passage et le passage d'une portion d'extrémité opposée de la bandelette à travers le deuxième orifice de passage, de telle sorte que le système de couplage de la bandelette s'étend d'un côté de l'objet et les portions d'extrémité s'étendent principalement de l'autre côté.

Selon une caractéristique avantageuse de l'invention, le système de couplage comprend une partie de la bandelette, appelée partie d'arrêt, qui est écartée des extrémités de la bandelette, et dont la largeur est supérieure à la largeur des portions extrémités de la bandelette et supérieure à la largeur des premier et deuxième orifices de passage ménagés dans l'objet.

L'invention concerne également un procédé d'extraction de l'objet d'un dispositif de conditionnement tel que décrit ci-dessus, ledit procédé comprenant les étapes suivantes :
- retrait du bouchon pour ouvrir le tube,
- saisie, par une main de l'opérateur, de la portion d'extrémité de la bandelette qui s'étend en saillie de l'ouverture du tube,
- saisie par une autre main, de l'objet couplé à la bandelette,
- découplage de l'objet par rapport à la bandelette.

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :
- la figure 1 est une vue d'un tube fermé par un bouchon, ledit tube contenant un implant couplé à une bandelette flexible, conformément à un premier mode de réalisation de l'invention ;
- la figure 2 est une vue d'un tube fermé par un bouchon, ledit tube contenant un implant couplé à une bandelette flexible, conformément à un deuxième mode de réalisation de l'invention ;
- la figure 3 est une vue d'un tube fermé par un bouchon, ledit tube contenant un implant couplé à une bandelette flexible, conformément à un troisième mode de réalisation de l'invention ;
- la figure 4 est une vue d'un tube fermé par un bouchon, ledit tube contenant un implant couplé à une bandelette flexible, conformément à un quatrième mode de réalisation de l'invention ;
- les figures 5 à 9 sont des vues montrant différentes étapes effectuées par un opérateur pour saisir la bandelette à laquelle est couplé l'implant, saisir l'implant et le découpler de la bandelette ;
- la figure 10 est une vue d'un tube fermé par un bouchon, ledit tube contenant un implant couplé à une bandelette flexible, conformément à un quatrième mode de réalisation de l'invention.
- la figure 11 est une vue de la section d'une bandelette flexible, conformément à un mode de réalisation de l'invention ;
- la figure 12 est une vue de la section d'une bandelette flexible, conformément à un autre mode de réalisation de l'invention ;
- la figure 13 est une vue en perspective d'une bandelette flexible, conformément à un autre mode de réalisation de l'invention selon lequel la bandelette se présente sous la forme d'une réglette munie de coulisseaux ;
- la figure 14 est une vue en perspective d'une bandelette flexible, conformément au mode de réalisation de la figure 13, à l'état fléchi pour récupérer l'objet précédemment tenu par les coulisseaux.

### DESCRIPTION DETAILLEE

Le concept de l'invention est décrit plus complètement ci-après avec référence aux dessins joints, sur lesquels des modes de réalisation du concept de l'invention sont montrés. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier. L'étendue de l'invention est par conséquent définie par les revendications jointes.

Une référence dans toute la description à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la description ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

En référence aux figures et comme rappelé ci-dessus, l'invention concerne un dispositif de conditionnement d'objet. Dans l'exemple illustré aux figures, ledit objet est un implant médical.

Ledit dispositif comprend un tube 1 qui est ouvert à une extrémité. Un bouchon 2 permet de refermer l'ouverture 10 du tube. Le tube contient un objet 5, tel qu'un implant médical.

Le dispositif de conditionnement comprend aussi un élément allongé flexible, appelé bandelette 3.

Ladite bandelette 3 comprend un système de couplage 4 permettant de coupler ledit objet 5 à ladite bandelette 3. Comme détaillé ci-après, l'objet est découplable par rapport à la bandelette.

La bandelette est flexible autour d'un axe parallèle au plan moyen de la bandelette et orthogonal à l'axe longitudinal de la bandelette.

La bandelette 3 est flexible de sorte que, à l'état non contraint, la bandelette présente une forme donnée sensiblement droite et, à l'état comprimé de la bandelette, la bandelette est comprimée à la manière d'une lame ressort, tout en étant rappelée dans sa forme initiale lorsque la contrainte qu'elle subit est supprimée.

A la manière d'une lame cintrable, la bandelette 3 peut être comprimée en étant maintenue sous tension par le bouchon et peut reprendre sa forme initiale sensiblement droite à l'état retiré du bouchon, c'est-à-dire lorsqu'elle ne subit plus de contrainte.

La bandelette 3 est configurée avec le tube 1 pour prendre une première position dans laquelle la bandelette 3 est maintenue comprimée à l'intérieur du tube 1 par le bouchon 2. Dans cette première configuration, la bandelette 3 présente une première extrémité 310 en contact d'appui avec le fond du tube 1 opposé à ladite ouverture 10. La bandelette 3 est aussi configurée avec le tube 1 pour prendre une deuxième position dans laquelle, le bouchon 2 étant retiré, la bandelette 3 n'est plus contrainte par le bouchon et présente une deuxième extrémité 320, opposée à ladite première extrémité 310, qui s'étend en saillie de l'ouverture 10 du tube 1.

En particulier, ladite bandelette 3 est de longueur supérieure à la longueur du tube 1 de sorte que, à l'état retiré du bouchon et donc à l'état non contraint, ladite bandelette présente une extrémité qui s'étend en saillie de l'ouverture 10 du tube 1.

Avantageusement, le tube 1 est translucide ou transparent. Préférentiellement, la bandelette 3 est aussi translucide ou transparente. Ainsi, l'utilisateur peut aisément et rapidement identifier l'objet contenu dans le tube.

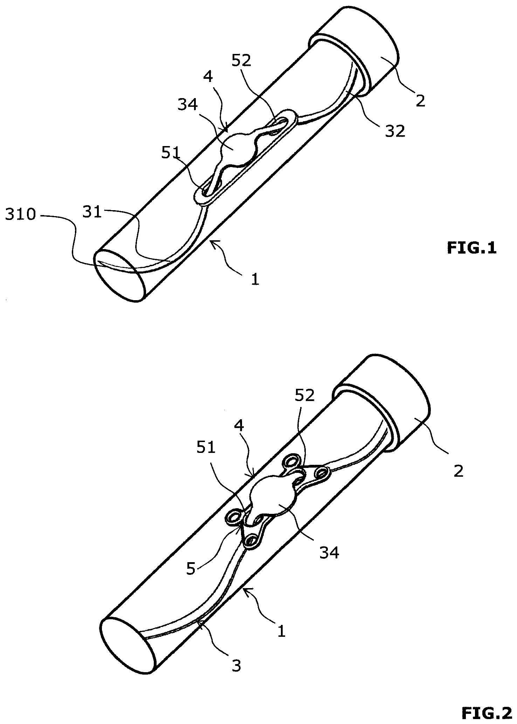

Dans chacun des modes de réalisation illustrés aux figures 1 à 9, un premier orifice 51 et un deuxième orifice 52 sont ménagés dans l'objet 5 pour permettre le passage d'une portion d'extrémité 31 de la bandelette 3 à travers le premier orifice de passage 51 et le passage d'une portion d'extrémité 32 opposée de la bandelette 3 à travers le deuxième orifice 52 de passage, de telle sorte que le système de couplage 4 de la bandelette 3 s'étend d'un côté de l'objet 5, c'est-à-dire d'un côté de orifices, et les portions d'extrémité 31, 32 s'étendent principalement de l'autre côté.

La première, respectivement deuxième, portion d'extrémité 31, 32, appelée premier, respectivement deuxième, brin, peut être définie comme la portion de la bandelette qui s'étend entre la première, respectivement deuxième, extrémité 310, 320 de la bandelette et le système de couplage 4 de l'objet. Le système de couplage 4 de l'objet peut être réalisé de différentes manières comme détaillé ci-après.

Selon les modes de réalisation illustrés aux figures 1 et 2, le système de couplage 4 comprend une partie 34 de la bandelette 3, appelée partie d'arrêt, qui est écartée des extrémités 310, 320 de la bandelette, et dont la largeur est supérieure à la largeur des portions extrémités 31, 32 de la bandelette 3. La largeur de la partie d'arrêt est aussi supérieure à la largeur des premier et deuxième orifices 51, 52 de passage ménagés dans l'objet 5.

Comme illustré dans les modes de réalisation des figures 1 et 2, ladite partie d'arrêt 34 forme une zone d'arrêt de l'objet 5 le long la bandelette 3. En effet la largeur de la partie d'arrêt 34 est supérieure à la largeur correspondante, c'est-à-dire prise suivant la même direction, des orifices de passage 51, 52 ménagés dans l'objet, ce qui permet de former une butée d'arrêt de glissement de la bandelette 3 selon l'axe longitudinal de la bandelette par rapport à l'objet 5 dans un sens et dans l'autre.

Dans l'exemple illustré à la figure 1, l'objet est un exemple de plaque chirurgicale de fixation osseuse (arthrodèse, compression métatarso-phalangienne ...) qui se présente sous le forme d'une pièce oblongue dans laquelle sont ménagés les deux orifices traversants 51, 52. Selon ce mode de réalisation, lesdits orifices traversants 51, 52 sont oblongs et situés à proximité des extrémités opposées de ladite pièce.

Dans l'exemple illustré à la figure 2, l'objet est un exemple de plaque chirurgicale de fixation osseuse qui se présente sous le forme d'une pièce munie de quatre oreilles disposées aux quatre coins de la pièce. Ladite pièce présente aussi les deux orifices traversants 51, 52 situés à proximités des extrémités opposées de ladite pièce.

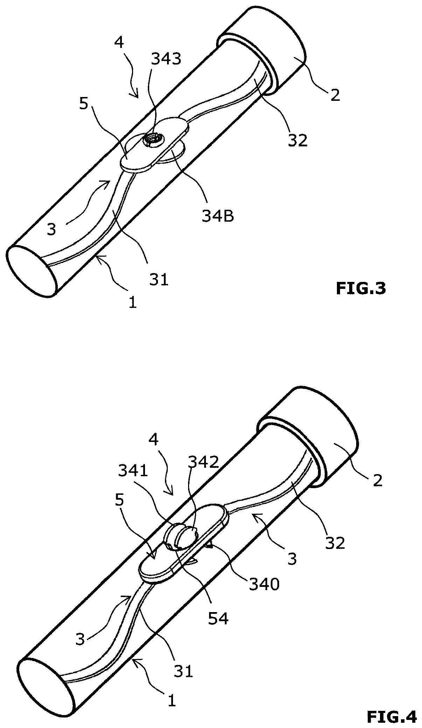

Selon le mode de réalisation illustré à la figure 3, dans lequel l'objet 5 est un exemple de plaque chirurgicale de fixation osseuse, le système de couplage 4 comprend un élément 343 encliquetable avec un orifice ménagé dans l'objet 5. Autrement dit, ledit élément 343 est déformable élastiquement et configuré pour pouvoir se déformer en se rétractant au passage dudit orifice ménagé dans l'objet 5 et pour pouvoir reprendre sa forme après traversée de l'orifice.

Avantageusement, ledit système de couplage comprend aussi une portion élargie 34B de la bandelette, située de préférence au milieu de la bandelette, qui forme une base sur laquelle est située ledit élément encliquetable. Comme illustré à la figure 3, ledit élément encliquetable 343 se présente sous la forme d'un champignon dont le chapeau est fendu pour permettre le passage par déformation élastique à travers l'orifice de l'objet 5.

Selon le mode de réalisation illustré à la figure 4, dans lequel l'objet est un exemple de plaque chirurgicale de fixation osseuse, ledit système de couplage 4 comprend une portion élargie de la bandelette, située de préférence au milieu de la bandelette 3, qui forme une base 340. Deux oreilles 341, 342 sont formées par découpe de ladite base 340. Lesdites oreilles 341, 342 sont apte à être redressées par rapport à ladite base 340, en étant ramenées l'une contre l'autre, pour être passées à travers un orifice 54 ménagé dans l'objet. Lesdites oreilles 341, 342 sont aptes à reprendre une position écartée l'une de l'autre après la traversée de l'orifice 54 de l'objet, dans laquelle la possibilité de retrait de l'objet 5 est limitée.

Comme illustré à la figure 4, lesdites oreilles présentent des encoches, formant des dents qui s'opposent au retrait des oreilles 341, 342 à travers l'orifice 54 de l'objet 5, ce qui empêche un retrait de l'objet 5 par rapport à la bandelette 3 en l'absence de déformation élastique imposée par l'opérateur.

Selon un mode de réalisation non illustré aux figures, on peut prévoir que ledit système de couplage 4 comprend deux fentes parallèles écartées ménagées à travers la bandelette 3 pour former un passant d'insertion d'un objet, tel qu'une vis, selon une direction d'introduction perpendiculaire aux fentes.

Avantageusement, la forme du tube 1 et celle de la bandelette 3 sont configurées pour limiter ou empêcher la rotation de la bandelette 3 par rapport au tube 1. Une telle configuration permet de maintenir l'objet 5 dans une position donnée suspendue en l'air, sans risque de dégradation de l'objet par des chocs contre la paroi du tube.

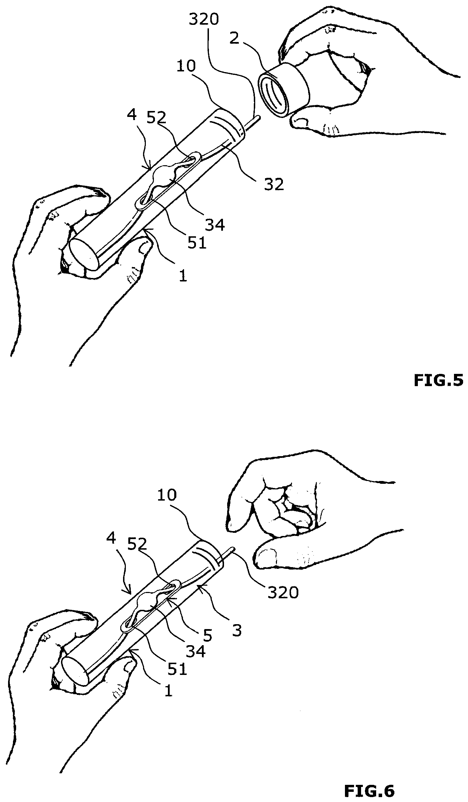

Les figures 5 à 9 illustrent différentes étapes permettant d'extraction d'un objet 5 d'un dispositif de conditionnement, tel que celui présenté à la figure 1.

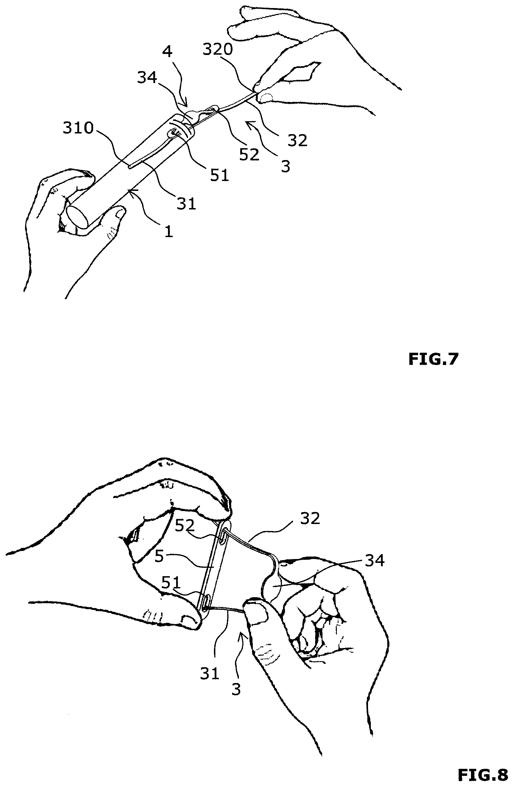

Comme illustré à la figure 5, l'opérateur retire le bouchon 2 pour ouvrir le tube 1, puis saisit par une main l'extrémité 320 de la bandelette qui s'étend en saillie de l'ouverture 10 du tube 2 (figure 6) pour la sortir du tube (figure 7) avec l'objet 5 qui lui est attaché. L'opérateur peut alors saisir, par son autre main, l'objet 5 couplé à la bandelette 3 et le découpler en retirant la bandelette (figure 8).

Dans les exemples illustrés aux figures 1 et 2, le découplage s'effectue en tirant sur la portion élargie 34 pour écarter la portion élargie de la bandelette par rapport à l'objet et faire sortir les extrémités 310, 320 de la bandelette par les orifices 51, 52 de l'objet 5.

Selon le mode de réalisation illustré à la figure 3, le découplage s'effectue en pressant sur l'élément 340 pour ramener ses deux portions vers la fente dudit élément et le faire passer à travers l'orifice de l'objet en tirant sur la portion élargie 34B pour écarter la portion élargie de la bandelette par rapport à l'objet et faire sortir les extrémités 310, 320 de la bandelette par les orifices 51, 52 de l'objet 5.

De manière similaire, selon le mode de réalisation illustré à la figure 4, le découplage s'effectue en ramenant les deux oreilles 341, 342 l'une vers l'autre pour les faire passer à travers l'orifice de l'objet en tirant sur la portion élargie 340 pour écarter la portion élargie 340 de la bandelette 3 par rapport à l'objet et faire sortir les extrémités 310, 320 de la bandelette par l'orifice 54 de l'objet 5.

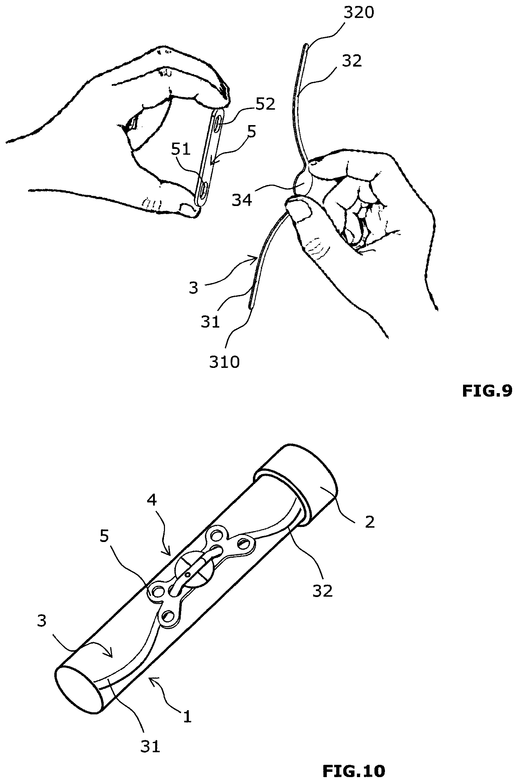

La figure 10 illustre un autre mode de réalisation de l'invention pour lequel la bandelette est réalisée en deux parties. Les deux parties sont assemblables et désassemblables l'une par rapport à l'autre de préférence par encliquetage. La zone de liaison entre les deux parties se situe de préférence à mi-longueur de la bandelette. Le fait de réaliser la bandelette en deux parties, permet, lorsque les orifices de passage de l'objet sont abrasifs, de retirer l'objet de la bandelette en la désassemblant et ainsi de ne pas avoir à tirer la bandelette sur toute sa longueur avec des frottements contre les orifices de passage qui pourraient générer des débris. En désassemblant les deux parties de la bandelette, l'appui de la bandelette sur les orifices de passage est réduit.

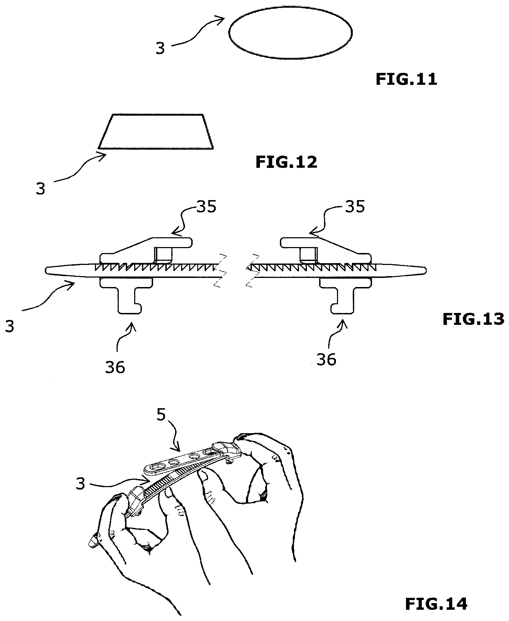

Comme illustré à la figure 11, la bandelette peut être de section elliptique, par exemple obtenue par injection en un élastomère thermoplastique tel que du SEBS : polystyrène-b-poly(éthylène-butylène)-b-polystyrène. La bandelette peut aussi être de section trapézoïdale (figure 12), par exemple obtenue par découpe d'une feuille en un matériau tel que du PET-G (Polyethylene terephthalate glycol-modified en anglais). D'autres formes de section sont envisageables.

Selon un mode de réalisation particulier illustré à la figure 13 et à la figure 14, la bandelette 3 se présente sous la forme d'une réglette munie de coulisseaux 35. Avantageusement, la réglette est aussi munie d'éléments de couplage 36 permettant le couplage de la réglette avec l'objet 5 lorsque celui-ci présente des orifices dans lesquels peuvent s'engager lesdits éléments de couplage. Avantageusement, lesdits éléments de couplage 36 se présentent sous la forme de plots situés du côté d'une face de la réglette, opposé aux parties actives des coulisseaux 35. Lesdits éléments de couplage 36 sont apte à s'engager à travers des orifices de l'objet.

Les coulisseaux 35 sont déplaçables l'un vers l'autre le long des crans de la réglette pour enserrer l'objet 5. Les crans sont configurés avec les coulisseaux pour ne permettre leur déplacement relatif que dans le sens d'un rapprochement l'un de l'autre.

Dans l'exemple illustré aux figures, les plots 36 sont portés par les coulisseaux 35. Ainsi, les coulisseaux 35 ont chacun d'un côté une partie active en forme de berceau pour attraper l'objet par l'extérieur et de l'autre des tétons ou plot 36 pour le maintenir par ses orifices de fixation ou par ses zones concaves.

Une telle bandelette munie de coulisseaux telle qu'illustrée aux figures 13 et 14 permet de proposer un mode de coincement de l'objet par l'extérieur dudit objet. Ainsi, une fois le bouchon du tube ouvert et une fois la bandelette 3 sortie du tube, l'objet 5 tenu par les parties actives des coulisseaux ou par les plots, peut être aisément récupéré par flambage (sans friction) de la réglette comme illustré à la figure 14.

L'invention n'est pas limitée aux modes de réalisation illustrés dans les dessins. En conséquence, il doit être entendu que, lorsque les caractéristiques mentionnées dans les revendications annexées sont suivies par des signes de référence, ces signes sont inclus uniquement dans le but d'améliorer l'intelligibilité des revendications et ne sont nullement limitatifs de la portée des revendications.

De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Dispositif de conditionnement d'objet comprenant :
- un tube (1) qui est ouvert (10) à une extrémité ;
- un bouchon (2) permettant de refermer ladite ouverture (10) du tube ;
**caractérisé en ce que** le dispositif de conditionnement comprend un élément allongé flexible, appelé bandelette (3),
ladite bandelette (3) étant de longueur supérieure à la longueur du tube (1), ladite bandelette (3) comprenant un système de couplage (4) permettant de coupler un objet (5), tel qu'un implant médical, à ladite bandelette (3),
la bandelette (3) étant configurée avec le tube (1) pour prendre :
- une première position dans laquelle la bandelette (3) est maintenue comprimée à l'intérieur du tube (1) par le bouchon (2), ladite bandelette (3) présentant une première extrémité (310) en contact d'appui avec le fond du tube (1) opposé à ladite ouverture (10),
- une deuxième position dans laquelle, le bouchon (2) étant retiré, la bandelette (3) présente une deuxième extrémité (320), opposée à ladite première extrémité (310), qui s'étend en saillie de l'ouverture (10) du tube (1).

2. Dispositif selon la revendication 1, **caractérisé en ce que** le tube (1) est translucide ou transparent.

3. Dispositif selon l'une des revendications précédentes, **caractérisé en ce que** le système de couplage (4) comprend un élément (343) encliquetable avec un orifice ménagé dans l'objet (5).

4. Dispositif selon l'une des revendications précédentes, **caractérisé en ce que** ledit système de couplage (4) comprend une portion élargie de la bandelette, située de préférence au milieu de la bandelette (3), qui forme une base (340), deux oreilles (341, 342) étant formées par découpe de ladite base (340), lesdites oreilles (341, 342) étant aptes à être redressées par rapport à ladite base (340), en étant ramenées l'une contre l'autre, pour être passées à travers un orifice (54) ménagé dans l'objet, lesdites oreilles (341, 342) étant aptes à reprendre une position écartée l'une de l'autre après la traversée de l'orifice (54) de l'objet, dans laquelle la possibilité de retrait de l'objet (5) est limitée.

5. Dispositif selon l'une des revendications précédentes, **caractérisé en ce que** la bandelette (3) se présente sous la forme d'une réglette présentant une série de crans et le système de couplage (4) comprend deux coulisseaux (35) rapprochables l'un de l'autre le long de la réglette pour enserrer l'objet, les crans étant configurés pour empêcher un déplacement à écartement desdits coulisseaux l'un par rapport à l'autre.

6. Dispositif selon l'une des revendications précédentes, **caractérisé en ce que** la forme du tube (1) et celle de la bandelette (3) sont configurées pour limiter ou empêcher la rotation de la bandelette (3) par rapport au tube (1).

7. Dispositif selon l'une des revendications précédentes, **caractérisé en ce que** ledit dispositif comprend ledit objet (5), ledit objet (5) étant couplé à la bandelette (3) et contenu dans le tube (1).

8. Dispositif la revendication 7, **caractérisé en ce qu'**un premier orifice (51) et un deuxième orifice (52) sont ménagés dans l'objet (5) pour permettre le passage d'une portion d'extrémité (31) de la bandelette (3) à travers le premier orifice de passage (51) et le passage d'une portion d'extrémité (32) opposée de la bandelette (3) à travers le deuxième orifice (52) de passage, de telle sorte que le système de couplage (4) de la bandelette (3) s'étend d'un côté de l'objet (5) et les portions d'extrémité (31, 32) s'étendent principalement de l'autre côté.

9. Dispositif selon la revendication 8, **caractérisé en ce que** le système de couplage (4) comprend une partie (34) de la bandelette (3), appelée partie d'arrêt, qui est écartée des extrémités (310, 320) de la bandelette, et dont la largeur est supérieure à la largeur des portions extrémités (31, 32) de la bandelette (3) et supérieure à la largeur des premier et deuxième orifices (51, 52) de passage ménagés dans l'objet (5).

10. Procédé d'extraction de l'objet (5) d'un dispositif de conditionnement conforme à l'une des revendications 7 à 9, ledit procédé comprenant les étapes suivantes :
- retrait du bouchon (2) pour ouvrir le tube (1),
- saisie, par une main de l'opérateur, de la portion d'extrémité (32) de la bandelette qui s'étend en saillie de l'ouverture (10) du tube (2),
- saisie par une autre main, de l'objet (5) couplé à la bandelette (3),
- découplage de l'objet (5) par rapport à la bandelette (3).

## Patentansprüche

1. Vorrichtung zum Verpacken eines Objekts, umfassend:
- ein Rohr (1), das an einem Ende offen (10) ist;
- einen Verschluss (2), der erlaubt, die Öffnung (10) des Rohrs zu verschließen;
**dadurch gekennzeichnet, dass** die Verpackungsvorrichtung ein flexibles längliches Element, Band (3) genannt, umfasst,
wobei das Band (3) länger als die Länge des Rohrs (1) ist, wobei das Band (3) ein Kopplungssystem (4) umfasst, das erlaubt, ein Objekt (5) wie ein medizinisches Implantat an das Band (3) zu koppeln,
wobei das Band (3) mit dem Rohr (1) ausgelegt ist, um einzunehmen:
- eine erste Position, in welcher das Band (3) komprimiert im Inneren des Rohrs (1) durch den Verschluss (2) gehalten wird, wobei das Band (3) ein erstes Ende (310) in Stützkontakt mit dem Boden des Rohrs (1) gegenüber der Öffnung (10) aufweist,
- eine zweite Position, in welcher, bei entferntem Verschluss (2), das Band (3) ein zweites Ende (320) gegenüber dem ersten Ende (310) aufweist, das sich aus der Öffnung (10) des Rohrs (1) herausragend erstreckt.

2. Vorrichtung nach Anspruch 1, **dadurch gekennzeichnet, dass** das Rohr (1) durchscheinend oder durchsichtig ist.

3. Vorrichtung nach einem der vorangehenden Ansprüche, **dadurch gekennzeichnet, dass** das Kopplungssystem (4) ein Element (343) umfasst, das in ein in das Objekt (5) ausgearbeitetes Loch einrastbar ist.

4. Vorrichtung nach einem der vorangehenden Ansprüche, **dadurch gekennzeichnet, dass** das Kopplungssystem (4) einen verbreiterten Abschnitt des Bandes umfasst, der sich vorzugsweise in der Mitte des Bandes (3) befindet, der eine Basis (340) bildet, wobei zwei Ohren (341, 342) durch Schneiden der Basis (340) gebildet sind, wobei die Ohren (341, 342) imstande sind, in Bezug auf die Basis (340) aufgerichtet zu sein, indem sie zueinander gebracht werden, um durch ein in dem Objekt ausgearbeitetes Loch (54) geführt zu werden, wobei die Ohren (341, 342) imstande sind, nach dem Durchgang durch das Loch (54) des Objekts erneut eine voneinander beabstandete Position einzunehmen, in welcher die Möglichkeit des Entfernens des Objekts (5) begrenzt ist.

5. Vorrichtung nach einem der vorangehenden Ansprüche, **dadurch gekennzeichnet, dass** das Band (3) in Form eines Lineals vorliegt, das eine Reihe von Rasten aufweist, und das Kopplungssystem (4) zwei entlang des Lineals einander annäherbare Schieber (35) aufweist, um das Objekt einzuspannen, wobei die Rasten konfiguriert sind, um eine beabstandende Verlagerung der Schieber zueinander zu verhindern.

6. Vorrichtung nach einem der vorangehenden Ansprüche, **dadurch gekennzeichnet, dass** die Form des Rohrs (1) und die des Bandes (3) ausgelegt sind, um die Rotation des Bandes (3) in Bezug auf das Rohr (1) zu begrenzen oder zu verhindern.

7. Vorrichtung nach einem der vorangehenden Ansprüche, **dadurch gekennzeichnet, dass** die Vorrichtung das Objekt (5) umfasst, wobei das Objekt (5) an das Band (3) gekoppelt und in dem Rohr (1) enthalten ist.

8. Vorrichtung nach Anspruch 7, **dadurch gekennzeichnet, dass** ein erstes Loch (51) und ein zweites Loch (52) in dem Objekt (5) ausgearbeitet sind, um den Durchgang eines Endabschnitts (31) des Bandes (3) durch das erste Durchgangsloch (51) und den Durchgang eines gegenüberliegenden Endabschnitts (32) des Bandes (3) durch das zweite Durchgangsloch (52) zu erlauben, so dass sich das Kopplungssystem (4) des Bandes (3) auf einer Seite des Objekts (5) erstreckt und sich die Endabschnitte (31, 32) hauptsächlich auf der anderen Seite erstrecken.

9. Vorrichtung nach Anspruch 8, **dadurch gekennzeichnet, dass** das Kopplungssystem (4) einen Teil (34) des Bandes (3), bezeichnet als Stoppteil, umfasst, der von den Enden (310, 320) des Bandes beabstandet ist und der breiter als die Breite der Endabschnitte (31, 32) des Bandes (3) und breiter als die Breite des ersten und zweiten Durchgangslochs (51, 52) ist, die in dem Objekt (5) ausgearbeitet sind.

10. Verfahren zur Extraktion des Objekts (5) aus einer Verpackungsvorrichtung nach einem der Ansprüche 7 bis 9, wobei das Verfahren die folgenden Schritte umfasst:
- Entfernen des Verschlusses (2), um das Rohr (1) zu öffnen,
- Ergreifen, durch eine Hand des Bedieners, des Endabschnitts (32) des Bandes, der sich aus der Öffnung (10) des Rohrs (2) herausragend erstreckt,
- Ergreifen, durch eine andere Hand, des an das Band (3) gekoppelten Objekts (5),
- Lösen des Objekts (5) in Bezug auf das Band (3).

## Claims

1. A device for packaging an object, comprising:
- a tube (1) that is open (10) at one end;
- a cap (2) making it possible to close said opening (10) of the tube;
**characterized in that** the packaging device comprises a flexible elongated element, called strip (3), said strip (3) having a length greater than the length of the tube (1),
said strip (3) comprising a coupling system (4) making it possible to couple an object (5), such as a medical implant, to said strip (3),
the strip (3) being configured with the tube (1) to assume:
- a first position in which the strip (3) is kept compressed inside the tube (1) by the cap (2), said strip (3) having a first end (310) in bearing contact with the bottom of the tube (1) opposite said opening (10),
- a second position in which, the cap (2) being removed, the strip (3) has a second end (320), opposite the first end (310), that protrudes from the opening (10) of the tube (1).

2. The device according to claim 1, **characterized in that** the tube (1) is translucent or transparent.

3. The device according to one of the preceding claims, **characterized in that** the coupling system (4) comprises an element (343) snapping with an orifice arranged in the object (5).

4. The device according to one of the preceding claims, **characterized in that** said coupling system (4) comprises a wider portion of the strip, preferably located in the middle of the strip (3), which forms a base (340), two lugs (341, 342) being formed by cutting of said base (340), said lugs (341, 342) being able to be straightened relative to said base (340), while being brought one against the other, to be passed through an orifice (54) arranged in the object, said lugs (341, 342) being able to return to a position separated from one another after passing through the orifice (54) of the object, wherein the possibility of extraction of the object (5) is limited.

5. The device according to one of the preceding claims, **characterized in that** the strip (3) assumes the form of a graduated strip having a series of notches and the coupling system (4) comprises two slides (35) able to be brought closer to one another along the graduated strip to grip the object, the notches being configured to prevent a separating movement of said slides relative to one another.

6. The device according to one of the preceding claims, **characterized in that** the shape of the tube (1) and that of the strip (3) are configured to limit or prevent the rotation of the strip (3) relative to the tube (1).

7. The device according to one of the preceding claims, **characterized in that** said device comprises said object (5), said object (5) being coupled to the strip (3) and contained in the tube (1).

8. The device according to claim 7, **characterized in that** a first orifice (51) and a second orifice (52) are arranged in the object (5) to allow the passage of an end portion (31) of the strip (3) through the first passage orifice (51) and the passage of an opposite end portion (32) of the strip (3) through the second passage orifice (52), such that the coupling system (4) of the strip (3) extends on one side of the object (5) and the end portions (31, 32) extend primarily on the other side.

9. The device according to claim 8, **characterized in that** the coupling system (4) comprises a part (34) of the strip (3), called stop part, that is separated from the ends (310, 320) of the strip, and the width of which is greater than the width of the end portions (31, 32) of the strip (3) and greater than the width of the first and second passage orifices (51, 52) arranged in the object (5).

10. A method of extraction of the object (5) from a packaging device according to one of claims 7 to 9, said method comprising the following steps:
- removing the cap (2) in order to open the tube (1),
- grasping, by one hand of the operator, the end portion (32) of the strip that protrudes from the opening (10) of the tube (2),
- grasping, by another hand, the object (5) coupled to the strip (3),
- uncoupling the object (5) relative to the strip (3).
